(19) Europäisches Patentamt

European Patent Office

Office européen des brevets

(11) **EP 0 560 336 B1**

## (12) EUROPEAN PATENT SPECIFICATION

(45) Date of publication and mention
of the grant of the patent:
**06.05.1998 Bulletin 1998/19**

(51) Int Cl.[6]: **C12M 1/40**

(21) Application number: **93103871.5**

(22) Date of filing: **10.03.1993**

(54) **A biosensor including a catalyst made from phosphate**

Biosensor mit einem Katalysator aus Phosphat

Biocapteur contenant un catalysateur de phosphat

(84) Designated Contracting States:
**DE FR GB**

(30) Priority: **12.03.1992 JP 53377/92**
**11.09.1992 JP 242919/92**

(43) Date of publication of application:
**15.09.1993 Bulletin 1993/37**

(73) Proprietor: **MATSUSHITA ELECTRIC INDUSTRIAL CO., LTD.**
**Kadoma-shi, Osaka-fu, 571 (JP)**

(72) Inventors:
- **Yoshioka, Toshihiko**
**Osaka-shi, Osaka (JP)**
- **Fujisawa, Satoko, Matsushita Elec. Ind. Co., Ltd.**
**Moriguchi-shi, Osaka (JP)**
- **Miyahara, Mariko, Matsushita Elec. Ind. Co., Ltd.**
**Moriguchi-shi, Osaka (JP)**
- **Nankai, Shiro, Matsushita Elec. Ind. Co., Ltd.**
**Hirakata-shi, Osaka (JP)**

(74) Representative: **Schwabe - Sandmair - Marx**
**Stuntzstrasse 16**
**81677 München (DE)**

(56) References cited:
**DE-A- 2 247 608**

- **BIOCHEMISCHE ZEITSCHRIFT vol. 110, 1920, BERLIN DE pages 181 - 192 HANS MURSCHHAUSER 'Die Mutarotation der Dextrose in L sungen von sekund rem Natriumphosphat'**
- **BIOCHEMISCHE ZEITSCHRIFT vol. 117, 1921, BERLIN DE pages 215 - 226 HANS MURSCHHAUSER 'Drehungserscheinungen von Dextrose in terti rem Natriumphosphat'**
- **DATABASE WPIL Section Ch, Week 8906, Derwent Publications Ltd., London, GB; Class A96, AN 89-044172 & JP-A-63 317 757 (MATSUSHITA ELECTRIC INDUSTRIAL KK) 26 December 1988**

## Description

The present invention relates to a catalyst made from phosphate and a biosensor that can easily quantify a specific component in various sample liquids with accuracy and speed. More particularly, the present invention relates to a biosensor that can easily quantify a substrate such as sucrose and glucose in a sample liquid by using a specific catalytic action of an enzyme. The biosensor contains a reaction layer comprising a hydrophilic polymer layer. The invention is characterised by the claims in detail.

The optical rotation method, the colorimetric method, the reductimetry method and other methods using different kinds of chromatographies have been developed as methods for quantitative analysis of a substrate such as sucrose and glucose. However, none of these methods has sufficiently high accuracy because the specificity thereof against the substrate is not very high. Among these methods, the optical rotation method can be easily conducted but is significantly influenced by the operating temperature. Therefore, it is not appropriate for common use at home and the like.

Various types of biosensors utilizing the specific catalytic action of an enzyme have been recently developed. As an example of such a biosensor, a sucrose sensor in which an enzyme reaction step comprises an isomerization step of pyranose will now be described.

A known method for quantifying sucrose uses three enzymes, invertase (EC3.2.1.26; hereinafter referred to as INV), mutarotase (EC5.1.3.3; hereinafter referred to as MUT) and glucose oxidase (EC1.1.3.4; hereinafter referred to as GOD), and an oxygen electrode or a hydrogen peroxide electrode (F. Scheller and F. Schubert, "Biosensor", Elsevier, 1992).

According to this method, sucrose is quantified as follows: Sucrose contained in a sample liquid is hydrolyzed into $\alpha$-glucose and fructose by INV. Then, isomerization from the $\alpha$-glucose to the $\beta$-glucose is accelerated by MUT, and only the $\beta$-glucose is selectively oxidized by GOD. In the oxidation reaction by GOD in the presence of oxygen, oxygen is reduced to hydrogen peroxide. The amount of decreased oxygen is measured by the oxygen electrode, or the amount of increased hydrogen peroxide is measured by the hydrogen peroxide electrode. Since the amount of the decreased oxygen or the increased hydrogen peroxide is in proportion to a content of the sucrose in the sample liquid, the sucrose is quantified by using the amount of the decreased oxygen or the increased hydrogen peroxide.

A substrate other than sucrose is quantified in the same manner.

For example, when a substrate such as glucose-6-phosphate, maltose, lactose and cellulose is quantified, $\alpha$-glucose is obtained by hydrolyzing each substrate in a sample liquid with an enzyme such as alkaline phosphatase, maltase, $\beta$-galactosidase and cellulase, respectively. Then, isomerization from the $\alpha$-glucose to

$\beta$-glucose is accelerated by MUT. The substrate in each sample liquid is quantified in the same manner as described above with respect to sucrose.

MUT used in the above described method for accelerating the isomerization from $\alpha$-glucose to $\beta$-glucose is comparatively expensive. Therefore, an inexpensive quantifying method for a substrate has been desired.

Activity of the above-mentioned enzymes such as INV, MUT and GOD generally tend to be degraded by various factors, thereby decreasing the reliability of the sensor.

Moreover, the specific activity of each enzyme depends upon a hydrogen ion concentration (pH), and each enzyme exhibits the highest activity at different pH. Therefore, when a plurality of sample liquids having different pH are measured, the obtained sensor responses are likely to fluctuate. This problem is noticeable especially when the sample liquid to be quantified is not pretreated.

In DE-A-2 247 608, "Biochemische Zeitschrift", vol. 110, (1920), pages 181-192 and vol. 117, (1921), pages 215-226 a phosphate buffer is disclosed as a catalyst for isomerizing $\alpha$-glucose to $\beta$-glucose. In JP-A-63 317 757 (see Derwent abstract 89-044172) a reaction layer is taught which is formed on an electrode system the reaction layer consisting of a water-absorptive macromolecular and enzymes as a mixture. In the preceding abstract no indication of a phosphate compound is given which is present in enzyme solution.

The catalyst made from phosphate of this invention accelerates the following equilibrium reaction:

$$\alpha\text{-glucose} \leftrightarrows \beta\text{-glucose}$$

In one preferred embodiment, the phosphate is at least one selected from the group consisting of potassium dihydrogenphosphate, dipotassium hydrogenphosphate, disodium hydrogenphosphate and sodium dihydrogenphosphate.

Alternatively, the present invention provides a method for accelerating the following equilibrium reaction by using phosphate:

$$\alpha\text{-glucose} \leftrightarrows \beta\text{-glucose}$$

Preferably, the phosphate is at least one selected from the group consisting of potassium dihydrogenphosphate, dipotassium hydrogenphosphate, sodium dihydrogenphosphate and disodium hydrogenphosphate.

In one preferred embodiment, the equilibrium reaction is conducted within a range of pH 4.0 to 10.5.

In one further embodiment, a concentration of the phosphate in the equilibrium reaction is within a range of 0.01 M to 0.8 M.

The biosensor of the present invention comprises the above-mentioned catalyst, an enzyme for oxidizing β-glucose, and an electrode system for electrically measuring an oxidation by the enzyme.

The biosensor of the present invention comprises an electrically insulating substrate, an electrode system which is formed on the substrate and has a working electrode and a counter electrode, and a reaction layer provided at a position to affect the electrode system. The reaction layer comprises at least an enzyme, an electron acceptor and phosphate.

The reaction layer further comprises a hydrophilic polymer layer.

The reaction layer is made of a first layer comprising a hydrophilic polymer layer and a second layer laminated on the first layer. The enzyme, the electron acceptor and the phosphate are contained in the second layer.

In one embodiment, the enzyme includes glucose oxidase.

In one embodiment, the enzyme further includes one selected from the group consisting of invertase, alkaline phosphatase, maltase, β-galactosidase and cellulase.

In one embodiment, the enzyme further includes mutarotase.

In one embodiment, a pH buffer layer is provided above the insulating substrate.

In one embodiment, the reaction layer comprises a pH buffer.

The reaction layer is made of a first layer comprising a hydrophilic polymer layer and a second layer laminated on the first layer. The enzyme, the electron acceptor and the phosphate are contained in the second layer, and the second layer further comprises a pH buffer.

In one embodiment, the enzyme includes a combination of invertase and glucose oxidase.

In one embodiment, the pH buffer is one selected from the group consisting of potassium dihydrogenphosphate - dipotassium hydrogenphosphate, potassium dihydrogenphosphate - disodium hydrogenphosphate, sodium dihydrogenphosphate - dipotassium hydrogenphosphate, sodium dihydrogenphosphate - disodium hydrogenphosphate, citric acid - disodium hydrogenphosphate, citric acid - dipotassium hydrogenphosphate, citric acid - sodium citrate, citric acid - potassium citrate, potassium dihydrogencitrate - sodium hydroxide, sodium dihydrogencitrate - sodium hydroxide, sodium hydrogenmaleate - sodium hydroxide, potassium hydrogenphthalate - sodium hydroxide, succinic acid - sodium tetraborate, maleic acid - tris(hydroxymethyl) aminomethane,tris(hydroxymethyl) aminomethane - tris tris(hydroxymethyl)aminomethane hydrochloride, [N-(2-hydroxyethyl)piperazine-N'-2-ethanesulfonic acid] - sodium hydroxide, [N-tris(hydroxymethyl)methyl-2-aminoethanesulfonic acid] - sodium hydroxide, and [piperazine-N,N'-bis(2-ethanesulfonic acid)] - sodium hydroxide.

Thus, the biosensor of the present invention comprises phosphate as a catalyst for accelerating an equilibrium reaction between α-glucose and β-glucose; an enzyme for selectively oxidizing the β-glucose; an electron acceptor which is reduced by electrons generated in an oxidation reaction by the enzyme; and an electrode system for electrically measuring an amount of the reduced electron acceptor.

In one embodiment, the biosensor further comprises an enzyme for generating α-glucose by hydrolyzing a saccharide.

Thus, the invention described herein makes possible the advantages of (1) providing a biosensor which can easily quantify a specific substrate in a sample liquid with accuracy and speed; (2) providing an inexpensive biosensor which can easily quantify a substrate without using expensive MUT; (3) providing a biosensor with a high reliability; (4) providing a biosensor which can obtain a sensor response with high accuracy by including a buffer in a reaction layer so that an enzyme reaction can proceed within a determined range of pH after the reaction layer is dissolved in a sample liquid; (5) providing a biosensor which does not require a preadjustment of a hydrogen ion concentration of a sample liquid and in which the hydrogen ion concentration of the sample liquid can be made optimum in accordance with the kind of enzyme contained in a reaction layer; (6) providing a biosensor which can quantify saccharides in fruit, blood, lymphocyte and urine; and (7) providing a catalyst made from phosphate for accelerating an equilibrium reaction between α-glucose and β-glucose and an acceleration method using the same.

These and other advantages of the present invention will become apparent to those skilled in the art upon reading and understanding the following detailed description with reference to the accompanying figures.

Figure 1 is a sectional view of a sucrose sensor according to an example of a biosensor of the present invention.

Figure 2 is an exploded perspective view of the sucrose sensor of Figure 1 from which a reaction layer is removed.

Figure 3 is a graph showing a sensor response of the sucrose sensor of Figure 1.

Figure 4 is a sectional view of a sucrose sensor according to another example of the biosensor of the present invention.

Figure 5 is a sectional view of a sucrose sensor according to still another example of the biosensor of the present invention.

According to the present invention a catalyst is used which is made from phosphate for accelerating the following equilibrium reaction:

$$\alpha\text{-glucose} \rightleftarrows \beta\text{-glucose}$$

Such isomerization from α-glucose to β-glucose is accelerated conventionally by an enzyme such as MUT.

Whereas the isomerization can be accelerated by using phosphate instead of the enzyme in the present invention.

A method for accelerating the equilibrium reaction by using phosphate can be utilized in a biosensor described below or in organic synthesis.

When the above-mentioned method is used in a biosensor, isomerization of pyranose is accelerated by phosphate ions such as $PO_4^{3-}$, $HPO_4^{2-}$ and $H_2PO_4^-$, and therefore, it is possible to obtain a rapid sensor response. Moreover, since there is no need to use an enzyme such as MUT for accelerating the isomerization, an inexpensive biosensor with a high reliability can be provided. An enzyme such as MUT can be used together with phosphate to further accelerate the equilibrium reaction. When the enzyme for accelerating the isomerization is included in the biosensor, the measuring time can be further shortened.

Any phosphate which can generate a phosphate ion such as $PO_4^{3-}$, $HPO_4^{2-}$ and $H_2PO_4^-$ in a reaction system can be used in the present invention. Examples of the phosphate include potassium dihydrogenphosphate ($KH_2PO_4$), dipotassium hydrogenphosphate ($K_2HPO_4$), sodium dihydrogenphosphate ($NaH_2PO_4$), disodium hydrogenphosphate ($Na_2HPO_4$), calcium dihydrogenphosphate ($Ca(H_2PO_4)_2$), ammonium phosphate ($(NH_4)_3PO_4$) and diammonium hydrogenphosphate ($(NH_4)_2HPO_4$).

The preferred pH in the equilibrium reaction can be freely selected to be suitable for an enzyme used together with the phosphate. In order to attain a sufficient effect of the phosphate for accelerating the equilibrium reaction, the pH is preferably within a range of 4.0 to 10.5.

A preferable concentration of the phosphate used in the equilibrium reaction is 0.01 M to 0.8 M, and more preferably 0.1 M to 0.8 M. When the concentration of the phosphate is over 0.8 M, in a biosensor in which a reaction layer is provided over an electrode system as is described in Examples 1 to 6 below, the reaction layer can not keep its shape on the electrode system. When the concentration of the phosphate is below 0.01 M, the effect of the phosphate to accelerate the equilibrium reaction is not sufficient.

The biosensor according to the present invention will now be described in detail.

The biosensor of the present invention comprises an electrically insulating substrate, an electrode system which is formed on the substrate and includes a working electrode and a counter electrode, and a reaction layer provided at a position to affect the electrode system.

The insulating substrate is made from a synthetic resin plate such as a polyethylene terephthalate plate.

The electrode system including the working electrode and the opposed counter electrode can be provided on the substrate by a known method. For example, after forming leads on the substrate, the working electrode and the counter electrode are provided so as to be connected to each lead, respectively, and to be insulated from each other. The electrode system is not limited to a two-electrode system having only a working electrode and a counter electrode. A three-electrode system, including an additional reference electrode, can be used. Such a three-electrode system can attain more accurate measurement.

The reaction layer can be provided over or in the vicinity of the electrode system. The reaction layer is generally provided so as to cover the working electrode and the counter electrode.

The reaction layer comprises a first layer made of a hydrophilic polymer layer directly provided on the electrode system and a second layer laminated on the first layer and including at least an enzyme, an electron acceptor and phosphate.

Various hydrophilic polymers can be used to form the hydrophilic polymer layer. Examples of the hydrophilic polymer include carboxymethyl cellulose (hereinafter referred to as CMC), hydroxyethyl cellulose (hereinafter referred to as HEC), hydroxypropyl cellulose (hereinafter referred to as HPC), methyl cellulose, ethyl cellulose, ethyl hydroxyethyl cellulose, carboxymethyl ethyl cellulose, polyvinyl pyrrolidone, polyvinyl alcohol, gelatin or its derivatives, acrylic acid or its salts, methacrylic acid or its salts, starch or its derivatives, and maleic anhydride or its salts. Among the above, CMC, HEC, HPC, methyl cellulose, ethyl cellulose, ethyl hydroxyethyl cellulose and carboxymethyl ethyl cellulose are preferred.

The enzyme in the reaction layer can comprise a first enzyme for hydrolyzing a substrate (especially a saccharide) into glucose and a second enzyme such as glucose oxidase for oxidizing β-glucose. The first enzyme is not necessary when glucose is quantified as a substrate.

The kind of first enzyme contained in the reaction layer depends upon the kind of substrate to be quantified.

A typical enzyme is invertase in quantifying sucrose; alkaline phosphatase in quantifying glucose-6-phosphate; maltase in quantifying maltose; β-galactosidase in quantifying lactose; and cellulase in quantifying cellulose.

The reaction layer can further comprise conventional mutarotase as an enzyme.

For example, the following enzymes are preferable in the quantification of sucrose:

1) A combination of invertase and glucose oxidase; and

2) a combination of invertase, glucose oxidase and mutarotase.

For example, the following enzymes are preferable in the quantification of glucose:

1) Glucose oxidase; and

2) a combination of glucose oxidase and mutarotase.

The electron acceptor contained in the reaction layer is reduced by electrons generated in the oxidation reaction caused by the enzyme. Examples of the electron acceptor include potassium ferricyanide, p-benzoquinone, phenazinemethosulfate, methylene blue and ferrocene derivatives. One or two kinds of the above are used as the electron acceptor.

The enzymes and the electron acceptors contained in the reaction layer can be allowed to be dissolved in a supplied sample liquid. Alternatively, they can be immobilized on the substrate and the like so as not to be dissolved in a sample liquid.

The reaction layer can further comprise a pH buffer. In such a case, the pH buffer is preferably contained in the second reaction layer.

Examples of the pH buffer include potassium dihydrogenphosphate - dipotassium hydrogenphosphate, potassium dihydrogenphosphate - disodium hydrogenphosphate, sodium dihydrogenphosphate - dipotassium hydrogenphosphate, sodium dihydrogenphosphate - disodium hydrogenphosphate, citric acid - disodium hydrogenphosphate, citric acid - dipotassium hydrogenphosphate, citric acid - sodium citrate, citric acid - potassium citrate, potassium dihydrogencitratesodium hydroxide, sodium dihydrogencitrate - sodium hydroxide, sodium hydrogenmaleate - sodium hydroxide, potassium hydrogenphthalate - sodium hydroxide, succinic acid - sodium tetraborate, maleic acid - tris(hydroxymethyl) aminomethane, tris(hydroxymethyl) aminomethane - tris(hydroxymethyl)aminomethane hydrochloride, [N-(2-hydroxyethyl)piperazine-N'-2-ethanesulfonic acid] - sodium hydroxide, [N-tris(hydroxymethyl)methyl-2-aminoethanesulfonic acid] - sodium hydroxide, and [piperazine-N,N'-bis(2-ethane-sulfonic acid)] - sodium hydroxide.

The reaction layer is made of the first and the second layers. The second layer can be formed by dropping a mixed solution including at least phosphate, an enzyme and an electron acceptor on the first layer and drying it. When the mixed solution including phosphate, an enzyme and an electron acceptor is dropped on the first layer made from CMC and the like, the first layer is once dissolved in the mixed solution and mixed therein superficially. Since the reaction layer made of the first and the second layers is not stirred and the two layers are not uniformly mixed, the surface of the electrode system is covered only by the first layer. Therefore, the enzyme and the electron acceptor do not come into contact with the surface of the electrode system, thereby preventing a degradation and a fluctuation of the response of the biosensor caused by adsorption of protein to the surface of the electrode system.

When a saccharide in a sample liquid is quantified by using the biosensor of the present invention, the saccharide is hydrolyzed by the first enzyme to generate α-glucose, and isomerization from the α-glucose to β-glucose is accelerated by phosphate. The β-glucose is oxidized by the second enzyme. The electron acceptor is reduced by electrons transferred by the oxidation reaction caused by the enzyme. Then, the amount of the reduced electron acceptor is electrically measured.

Therefore, when a sample liquid is supplied to the biosensor, the reaction layer is dissolved in the sample liquid. Next, a determined period of time after the supply of the sample liquid, a determined voltage is applied between the working electrode and the counter electrode, and a current value after a determined period of time is measured. This current value is in proportion to a concentration of a substrate in the sample liquid. A number of sample liquids each including the substrate with a known concentration are previously measured for this current value to identify a relationship between the concentration and the current value. Then, the substrate in a sample liquid can be quantified by measuring a current value in the above-mentioned manner.

The concentration of a substrate in a sample liquid can be measured without using an electron acceptor. In this case, β-glucose is oxidized by an enzyme to generate gluconic acid, and at the same time, oxygen is reduced to generate hydrogen peroxide. The hydrogen peroxide is electrochemically oxidized to cause an oxidation current. Since the current value is in proportion to the concentration of the substrate, the concentration can be quantified by using the oxidation current value.

The biosensor of the present invention can be used as a sensor for measuring various kinds of substrates by selecting the kind of enzymes to be used. For example, by using invertase, alkaline phosphatase, maltase, β-galactosidase or cellulase as an enzyme, the sensor works as a sucrose sensor, a glucose sensor, a glucose-6-phosphate sensor, a maltose sensor, a lactose sensor or a cellulose sensor.

Thus, the present invention provides a biosensor which can easily quantify a substrate in various kinds of sample liquids with accuracy and speed. For example, a saccharide in fruit, blood, lymphocyte and urine can be quantified by using the biosensor of the present invention.

Examples

Throughout the drawings mentioned in the following description of the examples, the same element has a common reference numeral. Part of the description of the production procedure is omitted as occasion demands.

(Example 1)

As an example of the biosensor of the present invention, a sucrose sensor will be described in this example.

A production method for the sucrose sensor is as follows:

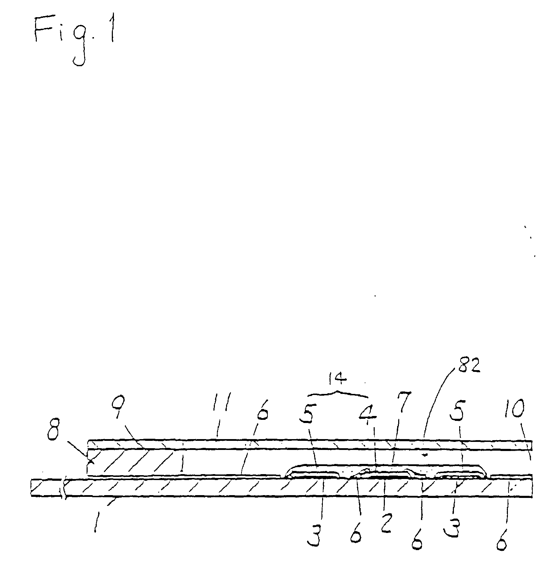

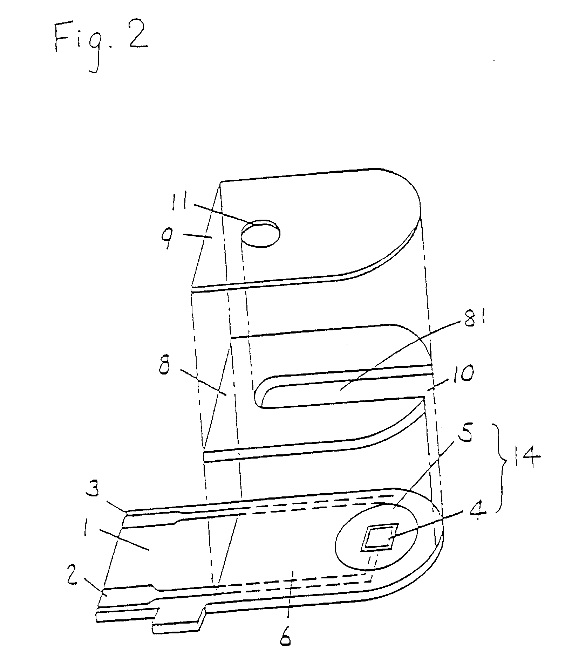

As are shown in Figures **1** and **2**, silver paste was printed by screen printing to form leads **2** and **3** on an insulating substrate **1** made from polyethylene terephthalate. Then, conductive carbon paste including a resin binder was printed on the substrate **1** to form a working electrode **4**. The working electrode **4** was in contact with the lead **2**. An insulating layer **6** was then formed by printing insulating paste on the substrate **1**. The insulating layer **6** covered the peripheral portion of the working electrode **4** so as to expose a fixed area of the working electrode **4**. The insulating layer 6 also covered part of the leads **2** and **3**.

Next, a counter electrode **5** was formed by printing conductive carbon paste including a resin binder on the insulating layer **6** so as to come in contact with the lead **3**.

An aqueous solution including 0.5 wt% CMC as a hydrophilic polymer was dropped on an electrode system **14** comprising the working electrode **4** and the counter electrode **5**, and dried to form a CMC layer. A mixed solution including INV and GOD as enzymes and potassium ferricyanide as an electron acceptor in a phosphate buffer (a mixture of 0.2 M of $KH_2PO_4$ and 0.2 M of $Na_2HPO_4$; pH 7.4) was dropped on the CMC layer, and dried in a warm-air drier at 50°C for 10 minutes to form a reaction layer **7**.

When the mixed solution including the phosphate, the enzymes and the electron acceptor was dropped on the CMC layer, the CMC layer made from a hydrophilic polymer was once dissolved to be superficially mixed with the enzymes and the like. However, the reaction layer **7** was not stirred and not mixed uniformly, and therefore, the surface of the electrode system **14** was covered only with the CMC layer.

Therefore, the enzymes and the electron acceptor did not come in contact with the surface of the electrode system **14**, thereby preventing a degradation and fluctuation of sensor response caused by adsorption of protein and the like to the surface of the electrode system **14**.

After forming the reaction layer **7** in the above described manner, a cover **9** and a spacer **8** were laminated to be adhered to the substrate **1** as shown in Figure **2** with dashed lines, thereby fabricating the sucrose sensor. The spacer **8** had a groove **81**. When the cover **9** and the spacer **8** were adhered to the substrate **1**, a passage **82** was formed by the groove **81**. One end of the passage **82** worked as a sample supply port **10** and the other works as an air port **11** having an opening on the cover **9**.

The reaction layer **7** can be formed on the cover **9**, the spacer **8** or the substrate **1** so as to face the passage **82**. The sample liquid supplied to the sensor fills the passage **82**, and can dissolve the reaction layer **7**.

Next, 3 µl of an aqueous solution of sucrose as a sample liquid was supplied through the sample supply port **10** of the sucrose sensor. The sample liquid rapidly reached the air port **11** through the passage **82** by capillarity, and dissolved the reaction layer **7**.

In order to supply a sample liquid more smoothly, a solution of lecithine in an organic solvent (for example, toluene) can be dropped so as to be spread over the reaction layer **7** and dried to form a lecithine layer before adhering the cover **9** and the spacer **8** to the substrate **1**.

Then, a determined period of time after the supply of the sample liquid to the sucrose sensor, a voltage (+0.5 V) was applied between the working electrode **4** and the counter electrode **5** via the leads **2** and **3**, and a current value of 5 seconds after the application was measured.

A plurality of sample liquids respectively having different sucrose concentrations were measured in the above-mentioned manner to obtain current values in proportion to the sucrose concentrations in the sample liquids.

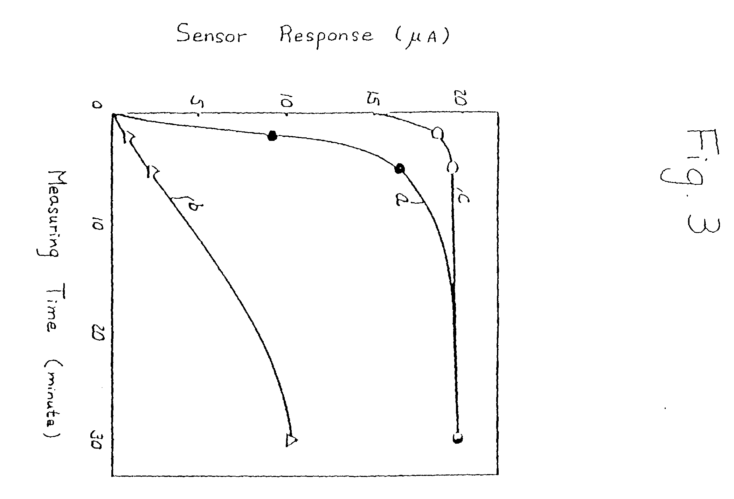

Figure **3** shows the relationship between the sensor response and the measuring time obtained by using the sucrose sensor in the above-mentioned manner.

In Figure **3**, a curve **a** shows results obtained by using the sucrose sensor of this example, a curve **b** shows results obtained by a sensor which has the same structure as the sensor of this example but has no phosphate, and a curve **c** shows results obtained by a sensor of Example 3 described below including MUT.

The results shown with the curves **a** and **b** show that the sucrose sensor of this example requires a shorter time to obtain a fixed sensor response than the sensor having no phosphate. The results shown with the curves **a** and **c** show that the sensor including MUT requires much shorter time to obtain a fixed sensor response than the sucrose sensor of this example.

The function of the sucrose sensor of this example is as follows:

Sucrose is hydrolyzed by INV to generate α-glucose. The α-glucose is rapidly isomerized to β-glucose by the catalytic function of the phosphate, and the β-glucose is oxidized by GOD. Potassium ferricyanide is reduced to potassium ferrocyanide by electrons transferred by the oxidation reaction by GOD. Then, an oxidation current of the generated potassium ferrocyanide is obtained by applying a voltage. This oxidation current corresponds to the concentration of sucrose, that is, a substrate in a sample liquid.

(Example 2)

As another example of the biosensor of the present invention, a sucrose sensor will be described in this example.

Leads **2** and **3**, an electrode system **14** comprising a working electrode **4** and a counter electrode **5**, and an insulating layer **6** were formed on an insulating substrate **1** made from polyethylene terephthalate in the same manner as in Example 1. Further, a CMC layer was formed on the electrode system **14**.

A mixed solution including INV, GOD, potassium ferricyanide and dipotassium hydrogenphosphate in a maleic acid - tris(hydroxymethyl)aminomethane buffer (pH 7.5) was dropped on the CMC layer, and dried in a warm-air drier to form a reaction layer **7**. A cover **9** and a spacer **8** were laminated to be adhered to the substrate I in the same manner as in Example 1 to fabricate the sucrose sensor.

Three µl of an aqueous solution of sucrose was supplied to the sucrose sensor through a sample supply port **10** in the same manner as in Example 1 to obtain an oxidation current value. The obtained value was in proportion to a concentration of the sucrose in the sample liquid.

(Example 3)

As still another example of the biosensor of the present invention, a sucrose sensor will be described in this example.

Leads **2** and **3**, an electrode system **14** comprising a working electrode **4** and a counter electrode **5** and an insulating layer **6** were formed on an insulating substrate **1** made from polyethylene terephthalate in the same manner as in Example 1.

Then, an aqueous solution including 0.5 wt% HEC was dropped on the electrode system **14** and dried to form an HEC layer. A mixed solution of INV, MUT, GOD, potassium ferricyanide and disodium hydrogenphosphate in an [N-(2-hydroxyethyl)piperazine-N'-2-ethanesulfonic acid] - sodium hydroxide buffer (pH 7.2) was dropped on the HEC layer, and dried in a warm-air drier to form a reaction layer **7**.

Ten µl of an aqueous solution of sucrose was supplied as a sample liquid to the sucrose sensor fabricated as above to dissolve the reaction layer **7**. A determined voltage was applied between the working electrode **4** and the counter electrode **5** after a determined period of time in the same manner as in Example 1, and an oxidation current was obtained.

The isomerization from $\alpha$-glucose to $\beta$-glucose is accelerated by functions of both MUT and phosphate ions. When both MUT and phosphate ions are contained in the reaction layer as they are in this example, the isomerization from $\alpha$-glucose to $\beta$-glucose can be further accelerated. As a result, a sensor response can be obtained in a shorter time.

(Example 4)

As still another example of the biosensor of the present invention, a glucose sensor will be described in this example.

Leads **2** and **3**, an electrode system **14** comprising a working electrode **4** and a counter electrode **5**, and an insulating layer **6** were formed on an insulating substrate **1** made from polyethylene terephthalate in the same manner as in Example 1.

Next, an aqueous solution of 0.5 wt% HPC was dropped on the electrode system **14** and dried to form an HPC layer. Then, a mixed solution of GOD, potassium ferricyanide and dipotassium hydrogenphosphate in a sodium dihydrogencitrate - sodium hydroxide buffer (pH 5.5) was dropped on the HPC layer, and dried in a warm-air drier at 50°C for 10 minutes to form a reaction layer **7**. A cover **9** and a spacer **8** were laminated to be adhered to the substrate **1** in the same manner as in Example 1 to fabricate a glucose censor.

Ten µl of an aqueous solution of glucose was supplied as a sample liquid to the glucose sensor fabricated as above to dissolve the reaction layer **7**. A determined period of time after the supply of the sample liquid, a determined voltage was applied between the working electrode **4** and the counter electrode **5** to measure an oxidation current.

GOD has a function to specifically oxidize only the $\beta$ compound among the isomers of glucose. In an aqueous solution of glucose, $\alpha$-glucose and $\beta$-glucose are equilibrated. In using the glucose sensor of this example, since the isomerization from $\alpha$-glucose to $\beta$-glucose is rapidly conducted by a function of the phosphate ions contained in the reaction layer, an oxidation current in proportion to a total amount of glucose in the sample liquid can be obtained in a short time.

Further, GOD used in this example has optimum pH around 5, and an enzyme reaction can be conducted around pH 5 by a buffering function of the buffer (sodium dihydrogencitrate - sodium hydroxide) contained in the reaction layer, whatever pH the sample liquid has. Therefore, an accurate measurement can be attained.

As the buffer, sodium dihydrogencitrate - NaOH (pH 5.5) can be replaced with citric acid - sodium citrate, citric acid - potassium citrate, potassium dihydrogencitrate - NaOH, sodium hydrogenmaleate - NaOH, potassium hydrogenphthalate - NaOH or succinic acid - sodium tetraborate, whose pH has been adjusted to be 5 to 6 in order to obtain the same effect as above.

(Example 5)

As still another example of the biosensor of the present invention, a sucrose sensor will be described in this example.

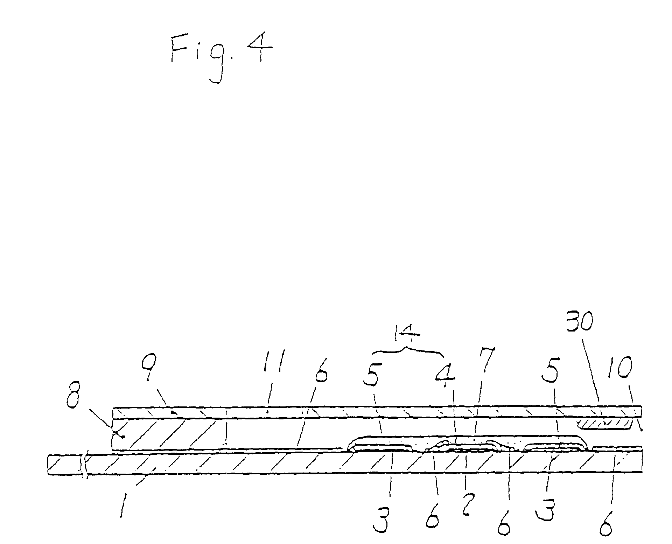

Figure **4** is a sectional view of the sucrose sensor of this example.

Leads **2** and **3**, an electrode system **14** comprising a working electrode **4** and a counter electrode **5**, and an insulating layer **6** were formed on an insulating substrate **1** made from polyethylene terephthalate in the same manner as in Example 1, and a CMC layer was formed on the electrode system **14**.

Next, a mixed solution of INV, MUT, GOD, potassium ferricyanide in a phosphate buffer (a mixture of 0.2 M of $KH_2PO_4$ and 0.2 M of $Na_2HPO_4$; pH 7.4) was dropped on the CMC layer, and dried in a warm-air drier at 50°C for 10 minutes to form a reaction layer **7**.

Then, a solution of CMC in a phosphate buffer (a mixture of 0.5 M of $KH_2PO_4$ and 0.5 M of $Na_2HPO_4$; pH 7.4) was dropped on a part on the inner surface of a cover **9** and dried to form a pH buffer layer **20**.

The cover **9** on which the pH buffer layer **20** was formed and a spacer **8** were laminated and adhered to the substrate **1** in the same manner as in Example 1 to fabricate the sucrose sensor.

Sucrose standard solutions having a fixed concentration and different pH from 3 to 7 were supplied as sample liquids to the sucrose sensor fabricated as above to obtain respective sensor responses in the same manner as in Example 1. The obtained oxidation values were in proportion to the sucrose concentration regardless of the pH of the sample liquids.

(Example 6)

As still another example of the biosensor of the present invention, a sucrose sensor will be described in this example.

Leads **2** and **3**, an electrode system **14** comprising a working electrode **4** and a counter electrode **5**, and an insulating layer **6** were formed on an insulating substrate **1** made from polyethylene terephthalate in the same manner as in Example 1. Further, a CMC layer was formed on the electrode system **14**.

Next, a mixed solution of INV, GOD, potassium ferricyanide and dipotassium hydrogenphosphate was dropped on the CMC layer, and dried to form a reaction layer **7**. Then, a cover **9** and a spacer **8** were laminated and adhered to the substrate **1** in the same manner as in Example 1 to fabricate the sucrose sensor.

Three μl of an aqueous solution of sucrose was supplied as a sample liquid through a sample supply port **10** to the sucrose sensor fabricated as above to obtain an oxidation current value. The obtained current value was in proportion to the sucrose concentration.

**Claims**

1. A biosensor comprising an electrically insulating substrate (1), an electrode system (14) which is formed on the substrate and has a working electrode (4) and a counter electrode (5), and a reaction layer (7) provided over the electrode system, wherein the reaction layer is made of a first layer comprising a hydrophilic polymer layer and a second layer laminated on the first layer, said second layer comprises

    a) a phosphate compound as a catalyst for accelerating an equilibrium reaction between α-glucose and β-glucose;

    b) an enzyme for selectively oxidizing the β-glucose; and

c) an electron acceptor which is reduced by electrons generated in an oxidation reaction by the enzyme.

2. A biosensor according to claim 1, wherein the enzyme includes glucose oxidase.

3. A biosensor according to claim 2, wherein the enzyme further includes one selected from the group consisting of invertase, alkaline phosphatase, maltase, β-galactosidase and cellulase.

4. A biosensor according to claim 1, wherein the enzyme further includes mutarotase.

5. A biosensor according to claim 1, wherein a pH buffer layer is provided above the insulating substrate.

6. A biosensor according to claim 1, wherein the reaction layer comprises a pH buffer.

7. A biosensor according to claim 6, wherein the pH buffer is comprised in the second layer of the reaction layer.

8. A biosensor according to claim 1, wherein the enzyme includes a combination of invertase and glucose oxidase.

9. A biosensor according to claim 6, wherein the pH buffer is one selected from the group consisting of potassium dihydrogenphosphate - dipotassium hydrogenphosphate, potassium dihydrogenphosphate - disodium hydrogenphosphate, sodium dihydrogenphosphate - dipotassium hydrogenphosphate, sodium dihydrogenphosphate - disodium hydrogenphosphate, citric acid - disodium hydrogenphosphate, citric acid - dipotassium hydrogenphosphate, citric acid - sodium citrate, citric acid - potassium citrate, potassium dihydrogencitrate - sodium hydroxide, sodium dihydrogencitrate - sodium hydroxide, sodium hydrogenmaleate - sodium hydroxide, potassium hydrogenphthalate - sodium hydroxide, succinic acid - sodium tetraborate, maleic acid - tris(hydroxymethyl)aminomethane, tris(hydroxymethyl)aminomethane - tris(hydroxymethyl) aminomethane hydrochloride, [N-(2-hydroxyethyl) piperazine-N'-2-ethane-sulfonic acid] - sodium hydroxide, [N-tris(hydroxymethyl)methyl-2-aminoethane acid] - sodium hydroxide, and [piperazine-N,N'-bis(2-ethane-sulfonic acid)] - sodium hydroxide.

**Patentansprüche**

1. Biosensor, umfassend ein elektrisch isolierendes Substrat (1), ein Elektrodensystem (14), das auf dem Substrat ausgebildet ist und eine Arbeitselektrode (4) und eine Gegenelektrode (5) aufweist, sowie eine über dem Elektrodensystem angeordnete Reaktionsschicht (7), wobei die Reaktionsschicht aus einer ersten Schicht mit einer hydrophilen Polymerschicht und einer auf die erste Schicht laminierten zweite Schicht besteht, die folgende Bestandteile aufweist:

a) einer Phosphatverbindung als Katalysator zur Beschleunigung einer Gleichgewichtsreaktion zwischen α-Glucose und β-Glucose;

b) einem Enzym für die selektive Oxidation der β-Glucose und

c) einem Elektronenakzeptor, der durch in einer Oxidationsreaktion durch das Enzym erzeugte Elektronen reduziert wird.

2. Biosensor nach Anspruch 1, in dem das Enzym Glucoseoxidase enthält.

3. Biosensor nach Anspruch 2, in dem das Enzym außerdem eine aus der Gruppe Invertase, alkalische Phosphatase, Maltase, β-Galactosidase und Cellulase ausgewählte Substanz enthält.

4. Biosensor nach Anspruch 1, in dem das Enzym außerdem Mutarotase enthält.

5. Biosensor nach Anspruch 1, in dem eine pH-Pufferschicht über dem isolierenden Substrat angeordnet ist.

6. Biosensor nach Anspruch 1, in dem die Reaktionsschicht einen pH-Puffer aufweist.

7. Biosensor nach Anspruch 6, in dem der pH-Puffer in der zweiten Schicht der Reaktionsschicht enthalten ist.

8. Biosensor nach Anspruch 1, in dem das Enzym eine Kombination aus Invertase und Glucoseoxidase aufweist.

9. Biosensor nach Anspruch 6, in dem der pH-Puffer aus der Gruppe Kaliumdihydrogenphosphat - Dikaliumhydrogenphosphat, Kaliumdihydrogenphosphat - Dinatriumhydrogenphosphat, Natriumdihydrogenphosphat - Dikaliumhydrogenphosphat, Natriumdihydrogenphosphat - Dinatriumhydrogenphosphat, Citronensäure - Dinatriumhydrogenphosphat, Citronensäure - Dikaliumhydrogenphosphat, Citronensäure -Natriumcitrat, Citronensäure - Kaliumcitrat, Kaliumdihydrogencitrat - Natriumhydroxid, Natriumdihydrogencitrat - Natriumhydroxid, Natriumhydrogenmaleat - Natriumhydroxid, Kaliumhydrogenphthalat - Natriumhydroxid, Bernsteinsäure - Natriumtetraborat, Maleinsäure - Tris(hydroxymethyl)aminomethan, Tris(hydroxymethyl)aminomethan - Tris(hydroxymethyl)aminomethanhydrochlorid, [N-(2-hydroxyethyl)piperazin-N'-2-ethansulfonsäure] - Natriumhydroxid, [N-Tris(hydroxymethyl)methyl-2-aminoethansulfonsäure] - Natriumhydroxid und [Piperazin-N,N'-bis(2-ethansulfonsäure] - Natriumhydroxid ausgewählt ist.

## Revendications

1. Biocapteur comprenant un substrat électriquement isolant (1), un système d'électrodes (14) qui est formé sur le substrat et comporte une électrode de travail (4) et une contre-électrode (5), et une couche réactionnelle (7) disposée au-dessus du système d'électrodes, dans lequel la couche réactionnelle est faite d'une première couche comprenant une couche de polymère hydrophile et d'une seconde couche stratifiée sur la première couche, ladite seconde couche comprend

a) un composé de phosphate en tant que catalyseur destiné à accélérer une réaction d'équilibre entre l'□-glucose et le □-glucose,
b) une enzyme destinée à oxyder de façon sélective le □-glucose, et
c) un accepteur d'électrons qui est réduit par les électrons générés dans une réaction d'oxydation par l'enzyme.

2. Biocapteur selon la revendication 1, dans lequel l'enzyme comprend la glucose-oxydase.

3. Biocapteur selon la revendication 2, dans lequel l'enzyme comprend en outre une enzyme choisie parmi le groupe constitué de l'invertase, de la phosphatate alcaline, de la maltase, de la □-galactosidase et de la cellulase.

4. Biocapteur selon la revendication 1, dans lequel l'enzyme comprend en outre la mutarotase.

5. Biocapteur selon la revendication 1, dans lequel une couche de tampon de pH est disposée au-dessus du substrat isolant.

6. Biocapteur selon la revendication 1, dans lequel la couche réactionnelle comprend un tampon de pH.

7. Biocapteur selon la revendication 6, dans lequel le tampon de pH est compris dans la seconde couche de la couche réactionnelle.

8. Biocapteur selon la revendication 1, dans lequel l'enzyme comprend une combinaison d'invertase et de glucose-oxydase.

9. Biocapteur selon la revendication 6, dans lequel le tampon de pH est l'un choisi parmi le groupe constitué des dihydrogénophosphate de potassium - hydrogénophosphate dipotassique, dihydrogénophosphate de potassium - hydrogénophosphate dissodique, dihydrogénophosphate de sodium - hydrogénophosphate dipotassique, dihydrogénophosphate de sodium - hydrogénophosphate dissodique, acide citrique - hydrogénophosphate dissodique, acide citrique - hydrogénophosphate dipotassique, acide citrique - citrate de sodium, acide citrique - citrate de potassium, dihydrogénocitrate de potassium - hydroxyde de sodium, dihydrogénocitrate de sodium - hydroxyde de sodium, hydrogénomaléate de sodium - hydroxyde de sodium, hydrogénophthalate de potassium - hydroxyde de sodium, acide succinique - tétraborate de sodium, acide maléïque - tris(hydroxyméthyl) aminométhane, tris(hydroxyméthyl) aminométhane - chlorydrate de tris(hydroxyméthyl) aminométhane, [acide N-(2-hydroxyéthyl) pipérazine - N'-2 éthanesulfonique] - hydroxyde de sodium, [acide N-tris(hydroxyméthyl) méthyl - 2 aminoéthanesulfonique] - hydroxyde de sodium, et [acide pipérazine - N, N' - bis (2 - éthanesulfonique)] - hydroxyde de sodium.

Fig. 1

EP 0 560 336 B1

Fig. 2

12

# Fig. 3

Fig. 4